# EUROPÄISCHE PATENTSCHRIFT

(11) **EP 0 663 957 B1**
(45) Veröffentlichungstag und Bekanntmachung des Hinweises auf die Patenterteilung: **27.11.1996**
(21) Anmeldenummer: 93920774.2
(22) Anmeldetag: 20.09.1993
(51) Int. Cl.: C12P 7/42, C12N 1/14

(54) **VERFAHREN ZUR FERMENTATIVEN HERSTELLUNG VON 2-HYDROXYPHENYLESSIGSÄURE**
FERMENTATIVE METHOD OF PREPARING 2-HYDROXYPHENYLACETIC ACID
PROCEDE DE PREPARATION FERMENTATIVE D'ACIDE 2-HYDROXYPHENYLACETIQUE

(30) Priorität: 29.09.1992 DE 4232522
(43) Veröffentlichungstag der Anmeldung: 26.07.1995
(73) Patentinhaber: BASF AKTIENGESELLSCHAFT, 67056 Ludwigshafen (DE)
(72) Erfinder: STAUDENMAIER, Horst, Ralf, D-6703 Limburgehof (DE); HAUER, Bernhard, D-6701 Fussgoenheim (DE); LADNER, Wolfgang, D-6701 Fussgoenheim (DE); MÜLLER, Ursula, D-6701 Fussgoenheim (DE); PRESSLER, Uwe, D-6701 Altrip (DE); MEYER, Joachim, D-6701 Maxdorf (DE)
(86) Internationale Anmeldenummer: EP9302541
(87) Internationale Veröffentlichungsnummer: WO9408029

(56) Entgegenhaltungen:
- PHYTOCHEMISTRY Bd. 7 , 1968 Seiten 1741 - 1742 J.K. FAULKNER ET AL. 'The metabolism of phenylacetic acid by Aspergillus niger' in der Anmeldung erwähnt
- FEMS MICROBIOLOGY LETTERS Bd. 5, Nr. 6 , Juni 1979 Seiten 427 - 430 M. SUGUMARAN ET AL. 'Microsomal hydroxylation of phenylacetic acid by Aspergillus niger'

## Beschreibung

Die Erfindung betrifft ein neues Verfahren zur Herstellung von 2-Hydroxyphenylessigsäure aus Phenylessigsäure.

Es ist bekannt, daß der Abbau von aromatischen Verbindungen durch Mikroorganismen häufig über hydroxylierte aromatische Zwischenprodukte verläuft, deren Ring anschließend unter Einführung weiterer Sauerstoffatome gespalten wird.

Ein Stoffwechselweg der Phenylessigsäure, der bei einer Reihe von Pilzen und Bakterien gefunden wird, verläuft zu 2-Hydroxyphenylessigsäure als erstem Produkt. In einer zweiten Monohydroxylierung wird dann eine weitere OH-Gruppe in Position 5 oder 6 des aromatischen Rings eingeführt. Diese Reaktionsfolge wurde z.B. bei Aspergillus niger und Pseudomonas fluorescens gefunden (Grazia Baggi et al., Styrene Catabolism by a Strain of Pseudomonas fluorescens, System. Appl. Microbiol. 4, 141 - 147, (1983); J.K. Faulkner and D. Woodcock, The Metabolism of Phenylacetic Acid by Aspergillus niger, Phytochemistry 7, 1741 - 1742, (1968); Fumiki Yoshizako et al., The Metabolism of Phenylacetic Acid by Aspergillus fumigatus ATCC 28282: Identification of 2,6-Dihydroxyphenylacetic Acid, Can. J. Microbiol. 23, 1140 - 1142 (1977)).

Diese Mikroorganismen sind für ein wirtschaftliches Herstellverfahren für 2-Hydroxyphenylessigsäure aus Phenylessigsäure nicht geeignet, da sie 2-Hydroxyphenylessigsäure nur als Stoffwechsel-Zwischenprodukt in kleinen Mengen bilden und sofort weiter abbauen.

Es bestand daher die Aufgabe, Mikroorganismen zu Verfügung zu stellen, die die oben erwähnten Nachteile nicht besitzen, und ein fermentatives Herstellungsverfahren für 2-Hydroxyphenylessigsäure aus Phenylessigsäure unter Verwendung dieser Mikroorganismen bereitzustellen.

Es wurde gefunden, daß für das eingangs beschriebene Verfahren Mikroorganismen geeignet sind, die einerseits die Fähigkeit besitzen, Phenylessigsäure in ortho-Position zu hydroxylieren, andererseits die gebildete 2-Hydroxyphenylessigsäure nicht weiter umsetzen, indem sie sie beispielsweise als Kohlenstoff-Quelle verwerten.

Solche Mikroorganismen erhält man zweckmäßigerweise durch Mutation von Wildstämmen, die die Fähigkeit zum Abbau von Phenylessigsäure über 2-Hydroxyphenylessigsäure besitzen. Als Wildstämme sind Bakterien, Aktinomyceten und Pilze geeignet. Besonders geeignete Vertreter finden sich beispielsweise in folgenden Gattungen:
Bacillus, Rhodococcus, Streptomyces, Absidia, Alternaria, Aspergillus, Beauveria, Botryodiplodia, Botrytis, Byssochlamys, Candida, Cephalosporium, Chaetomium, Cunninghamella, Curvularia, Dactylium, Drechslera, Epicoccum, Fusarium, Geotrichum, Gibberella, Gleophyllum, Gliocladium, Helminthosporium, Humicola, Hyphozyma, Metarrhizium, Microascus, Mucor, Neurospora, Paecilomyces, Penicillium, Phycomyces, Phyllosticta, Pythium, Rhizopus, Septoria, Sphaceloma, Trichoderma, Trichosporon, Trichurus, Verticillium.

Geeignete Mikroorganismen können leicht durch den in Beispiel 1 beschriebenen, einfachen Test identifiziert werden.

Von den geeigneten Mikroorganismen werden zweckmäßigerweise Mutanten erzeugt oder isoliert, die nicht mehr in der Lage sind, 2-Hydroxyphenylessigsäure abzubauen.

Zur Erzeugung solcher Mutanten können bekannte mikrobiologische Techniken eingesetzt werden. Zur Auslösung von Mutationen können alle gängigen Methoden verwendet werden wie die Anwendung von mutagenen Substanzen, z.B. Nitrosoguanidin, Ethylmethansulfonat, Natriumnitrit , oder die Einwirkung von elektromagnetischer Strahlung wie UV-, Gamma- oder Röntgenstrahlung. Weiterhin können zur Mutagenese auch transponierbare genetische Elemente verwendet werden. Zur Isolierung der Mutanten kann beispielsweise die Eigenschaft, auf Phenylessigsäure als einziger C-Quelle nicht mehr zu wachsen, benützt werden.

Es wurde ein neuer Pilz der Gattung Humicola oder Chaetomium gefunden, der unter der Hinterlegungsnummer DSM 7047 bei der Deutschen Sammlung von Mikroorganismen und Zellkulturen GmbH, Braunschweig, hinterlegt wurde.

Weiterhin wurde gefunden, daß der Pilz DSM 7047 besonders gut zur Hydroxylierung von aromatischen Verbindungen, insbesondere von aromatischen Säuren und Säurederivaten verwendet werden kann. Es wurde weiterhin gefunden, daß sich durch Züchtung des Pilzes DSM 7047 in Gegenwart von Phenylessigsäure ein vorteilhaftes Herstellungsverfahren für 2-Hydroxyphenylessigsäure aus Phenylessigsäure ergibt.

Der Pilz DSM 7047 wurde von CBS (Centraalbureau voor Schimmelcultures, Baarn, Niederlande) als Humicola fuscoatra Traaen bestimmt, während er von DSM als Chaetomium, vermutlich Chaetomium seminudum, bezeichnet wurde. Eine endgültige Zuordnung zu der Gattung Humicola oder Chaetomium ist derzeit nicht möglich. Der Stamm DSM 7047 zeichnet sich dadurch aus, daß er Phenylessigsäure in ortho-Position hydroxyliert, aber die entstandene 2-Hydroxyphenylessigsäure nicht metabolisiert.

Zur Prüfung, ob dies ein generelles Merkmal der Gattungen Humicola oder Chaetomium ist, wurden verschiedene Stämme dieser Gattungen analog Beispiel 1 getestet:

| | |
|---|---|
| Humicola brevispora | ATCC 28403 |
| Humicola brunnea | ATCC 22629 |
| Humicola fuscoatra | ATCC 22723 |
| Humicola fuscoatra | ATCC 22721 |
| Humicola fuscoatra | Li 664 |
| Humicola grisea | ATCC 22724 |
| Humicola grisea | Li 193 |
| Humicola parvispora | ATCC 22715 |
| Chaetomium alba-avenulum | Li 69 |
| Chaetomium globosum | ATCC 6205 |
| Chaetomium globosum | Li 70 |
| Chaetomium globosum | Li 444 |

Die mit Li bezeichneten Stämme stammen aus der Stammsammlung der landwirtschaftlichen Versuchsstation der BASF Aktiengesellschaft in Limburgerhof.

Bei allen diesen Stämmen konnte 2-Hydroxiphenylessigsäure in geringen Mengen nachgewiesen werden. Sie wurde jedoch stets wieder vollständig abgebaut.

Durch Mutagenese mit UV-Licht konnten aus DSM 7047 in einem Schritt Revertanten gewonnen werden, die Phenylessigsäure genauso abbauten wie die oben aufgelisteten Humicola- und Chaetomium-Wildtypstämme. Dadurch wurde gezeigt, daß es sich bei DSM 7047 um eine Mutante eines Phenylessigsäureabbauers handelt.

Das erfindungsgemäße Verfahren zur Herstellung von 2-Hydroxyphenylessigsäure aus Phenylessigsäure beinhaltet die Züchtung der geeigneten Mikroorganismen in einem üblichen Nährmedium. Geeignet sind Nährmedien, die Kohlenstoffquellen, Stickstoffquellen, anorganische Salze und gegebenenfalls geringe Mengen an Spurenelementen und Vitaminen enthalten.

Als Stickstoffquellen können anorganische bzw. organische Stickstoffverbindungen oder Materialien, die diese Verbindungen enthalten, verwendet werden. Beispiele sind Ammoniumsalze, Nitrate, Maisquellwasser, Bierhefeautolysat, Sojabohnenmehl, Weizengluten, Hefeextrakt, Hefe, Harnstoff und Kartoffelprotein.

Als Kohlenstoffquellen können beispielsweise Zucker wie Glucose, Polyole wie Glyzerin oder Fette wie Sojaöl verwendet werden. Beispiele für anorganische Salze sind die Salze von Kalzium, Magnesium, Mangan, Zink, Kupfer, Eisen und anderen Metallen. Als Anion der Salze ist besonders das Phosphation zu nennen. Gegebenenfalls werden dem Nährmedium Wachstumsfaktoren wie Biotin, Riboflavin und andere Vitamine zugesetzt.

Ein besonders gut geeignetes Nährmedium ist das Medium 2, das in Beispiel 2 beschrieben wird.

Dem Nährmedium wird Phenylessigsäure zugesetzt. Phenylessigsäure kann als freie Säure oder als Salz, bevorzugt als Alkali- oder Ammoniumsalz, dem Medium zugesetzt werden. Da die Löslichkeit von Salzen in wäßrigen Nährmedien im allgemeinen größer ist als die der freien Säure, wird das Natrium- oder Ammoniumsalz der Phenylessigsäure bevorzugt verwendet. Das entsprechende Salz kann zweckmäßigerweise durch Titration der Phenylessigsäure mit einer Base in situ hergestellt werden.

Die Phenylessigsäure wird in der Regel in solchen Mengen zugegeben, daß eine Konzentration von 0,5 bis 30 g, bevorzugt von 1 bis 20 g, pro Liter Nährmedium eingestellt wird.

Die Phenylessigsäure kann dem Nährmedium am Anfang der Züchtung des Mikroorganismus oder während der Züchtung in mehreren Portionen oder kontinuierlich zugegeben werden.

Außerdem ist es möglich, den gezüchteten Mikroorganismus nach beendetem Umsatz zurückzubehalten und ihn in frischem Nährmedium mit Phenylessigsäure weiterzuzüchten.

Diese Form der Wiederverwendung der Biomasse ist eine besonders vorteilhafte Ausgestaltung des erfindungsgemäßen Verfahrens, da dabei beispielsweise die Anzuchtszeit für die Biomasse eingespart wird.

Die Züchtung des Mikroorganismus bedarf keiner weiteren besonderen Bedingungen. So kann die Züchtungstemperatur in der Regel zwischen 20 und 40°C liegen, bevorzugt wird eine Temperatur zwischen 25 und 35°C gewählt.

Der pH-Wert des Fermentationsmediums wird während der Fermentation zwischen 3 und 9 festgehalten. Vorteilhaft sind pH-Werte zwischen 4 und 7. Eine Ansäuerung des Mediums während der Fermentation kann beispielsweise durch Zugabe einer Base wie Ammoniak kompensiert werden.

Die Fermentationszeiten betragen üblicherweise zwischen 1 und 10 Tagen, um eine maximale Anreicherung des Produkts im Fermentationsmedium zu erreichen.

Der Reaktionsumsatz kann leicht durch Probenentnahme und beispielsweise gaschromatographische Analyse festgestellt werden. Die Isolierung und Reinigung der 2-Hydroxyphenylessigsäure aus dem Nährmedium kann nach bekannten Methoden erfolgen. Zweckmäßigerweise trennt man die feste Biomasse vom Nährmedium ab, extrahiert den Wertstoff z. B. mit einem organischen Lösungsmittel, gegebenenfalls nach vorausgegangener Ansäuerung des Mediums, und isoliert den Wertstoff aus der extrahierten Phase.

Die Erfindung wird durch die folgenden Beispiele weiter veranschaulicht.

### Beispiel 1

Test von Mikroorganismen auf ortho-Hydroxylierung von Phenylessigsäure

Reinkulturen von Mikroorganismen aus öffentlichen Stammsammlungen oder aus Bodenisolaten (Drews: Mikrobiologisches Praktikum, 3. Auflage, Springer Verlag 1976, Seite 47-48) wurden in einem Test daraufhin überprüft, ob sie Phenylessigsäure spezifisch in ortho-Position hydroxylieren können.

Dazu wurden die Stämme von Agarplatten in Flüssigmedium mit Phenylessigsäure angeimpft. Es wurde folgendes Medium verwendet (M1-Medium):

| | |
|---|---|
| 10 | g/l Glucose |
| 5 | g/l Hefeextrakt (Difco) |
| 5 | g/l (NH₄)₂SO₄ |
| 0,5 | g/l MgSO₄ x 7H₂O |
| 0,05 | g/l MnSO₄ x 4H₂O |
| 2 | ml/l Spurenelementlösung |
| 1,5 | g/l KH₂PO₄ |
| 3,6 | g/l K₂HPO₄ |

Spurenelementlösung:

| | |
|---|---|
| 200 | mg/l Eisen(II)sulfat-1-hydrat |
| 10 | mg/l Zink(II)sulfat-4-hydrat |
| 3 | mg/l Manganchlorid-4-hydrat |
| 30 | mg/l Borsäure |
| 20 | mg/l Cobalt(II)chlorid-6-hydrat |
| 1 | mg/l Kupfer(II)chlorid-2-hydrat |
| 2 | mg/l Nickel(II)chlorid-6-hydrat |
| 3 | mg/l Natriummolybdat-2-hydrat |
| 500 | mg/l Ethylendiamintetraessigsäure (EDTA) |

Die Phenylessigsäure wurde als 25 %ige Lösung angesetzt, mit NaOH auf pH 7 titriert, autoklaviert und zu dem separat angesetzten Medium zugegeben.

250 ml-Erlenmeyerkolben wurden mit 30 ml M1-Medium mit 1 g/l Phenylessigsäure gefüllt, mit jeweils einem Mikroorganismenstamm von Agarplatten angeimpft und bei 25°C mit 180 U/min schüttelnd inkubiert. Nach 3, 7 und 10 Tagen wurde 1 ml des Kulturüberstandes abgenommen, mit 100 µl 5 N Salzsäure und 800 µl Essigsäureethylester versetzt und 15 s kräftig gemischt. 700 µl der Essigsäureethylesterphase wurden abgenommen und bei 50°C unter einem leichten Stickstoffstrom eingedampft. Der Rückstand wurde in 70 µl Essigsäureethylester gelöst und 50 µl davon in ein Probegläschen für die Gaschromatographie überführt. Dazu wurden 50 µl N-Methyl-N-trimethylsilyltrifluoracetamid (MSTFA) gegeben und gemischt. Die Proben wurden gaschromatographisch untersucht. Als Vergleich diente eine authentische Probe 2-Hydroxyphenylessigsäure.

Mit Stämmen aus folgenden Gattungen wurde die Bildung von 2-Hydroxyphenylessigsäure beobachtet:
Pilze:
Absidia
Alternaria
Aspergillus
Beauveria
Botryodiplodia
Botrytis
Byssochlamys
Candida
Cephalosporium
Chaetomium
Cunninghamella
Curvularia
Dactylium
Drechslera
Epicoccum
Fusarium
Geotrichum
Gibberella
Gleophyllum
Gliocladium
Helminthosporium
Humicola
Hyphozyma
Metarrhizium
Microascus
Mucor
Neurospora
Paecilomyces
Penicillium
Phycomyces
Phyllosticta
Pythium
Rhizopus
Septoria
Sphaceloma
Trichoderma
Trichosporon
Trichurus
Verticillium

Streptomyceten:
Streptomyces aureofaciens
Streptomyces hygroscopicus
Streptomyces kasugaensis
Streptomyces niveus
Streptomyces roseochromogenus
Streptomyces viridifaciens

Bakterien:
Bacillus
Rhodococcus

### Beispiel 2

### Umsetzung von Phenylessigsäure mit DSM 7047

Medium 2:
50 g/l Glucose
10 g/l Hefeextrakt (Difco)
10 g/l (NH₄)₂SO₄
0,5 g/l MgSO₄·7H₂O
3,6 g/l K₂HPO₄
1,5 g/l KH₂PO₄
10 ml/l Spurenelementlösung

Vorkultur: DSM 7047 wurde von einer Agarplatte in 150 ml Medium 2 mit 1 g/l Phenylessigsäure und zusätzlich 3 g/l Carbopol® 946 in einem 1 l-Erlenmeyerkolben angeimpft und 3 Tage bei 30°C und 180 U/min geschüttelt.

Mit dieser Vorkultur wurde ein 1,5 l-Fermenter mit Medium 2 und 5 g/l Phenylessigsäure angeimpft. Zusätzlich wurde 1 ml/l Pluriol P 2000 zur Verhinderung der Schaumentwicklung zugegeben.

Der Fermenter wurde auf 30°C temperiert, mit 1 l Luft pro Minute (= 0,66 vvm) begast und mit 600 U/min gerührt.

Nach 5 Tagen war die Phenylessigsäure vollständig zu 2-Hydroxyphenylessigsäure umgesetzt.

Die Zellen wurden durch Watte-Filtration abgetrennt und mit 100 ml Wasser nachgewaschen. Die zellfreie Kulturbrühe und das Waschwasser wurden vereinigt, mit HCl auf pH2 gestellt und zweimal mit dem gleichen Volumen tert-Butyl-methylether extrahiert.

Nach dem Eindampfen der organischen Phase bis zur Trockne wurde ein Rückstand von 8,2 g erhalten, der zu 98 % (ermittelt über GC-Analyse) aus 2-Hydroxyphenylessigsäure bestand. Die Struktur des Produkts wurde durch NMR bestätigt.

### Beispiel 3

### Verbesserte Fermentation von DSM 7047

Vorkultur und Animpfen des Fermenters wurde wie in Beispiel 2 durchgeführt; der pH-Wert des Fermentationsmediums sank während der Fermentation von 6,8 auf 5,6 ab. Ein weiteres Ansäuern wurde durch Zugabe von Ammoniak kompensiert. Sobald die Phenylessigsäure-Konzentration auf einen Wert zwischen 2,5 und 1 g/l abgesunken war, wurden viermal 1 g/l Phenylessigsäure zudosiert. Nach 7 Tagen war die Phenylessigsäure vollständig zu 2-Hydroxyphenylessigsäure umgesetzt.

## Patentansprüche

1. Verfahren zur Herstellung von 2-Hydroxyphenylessigsäure aus Phenylessigsäure, dadurch gekennzeichnet, daß man einen Mikroorganismus in Gegenwart von Phenylessigsäure züchtet, der Phenylessigsäure in ortho-Position hydroxyliert und die 2-Hydroxyphenylessigsäure nicht weiter umsetzt.

2. Verfahren nach Anspruch 1, dadurch gekennzeichnet, daß als Mikroorganismus ein Pilz der Gattung Humicola oder Chaetomium verwendet wird.

3. Verfahren nach Anspruch 1, dadurch gekennzeichnet, daß als Mikroorganismus der Pilz DSM 7047 verwendet wird.

4. Pilz der Gattung Humicola oder Chaetomium, welcher unter der Hinterlegungsnummer DSM 7047 hinterlegt ist.

5. Verwendung eines Pilzes gemäß Anspruch 4 zur Hydroxylierung von aromatischen Verbindungen.

## Claims

1. A process for preparing 2-hydroxyphenylacetic acid from phenylacetic acid, which comprises cultivating in the presence of phenylacetic acid a microorganism which hydroxylates phenylacetic acid in the ortho position and does not transform 2-hydroxyphenylacetic acid further.

2. A process as claimed in claim 1, wherein a fungus of the genus Humicola or Chaetomium is used as microorganism.

3. A process as claimed in claim 1, wherein the fungus DSM 7047 is used as microorganism.

4. A fungus of the genus Humicola or Chaetomium which is deposited under deposit number DSM 7047.

5. The use of a fungus as claimed in claim 4 to hydroxylate aromatic compounds.

## Revendications

1. Procédé de fabrication de l'acide 2-hydroxyphénylacétique à partir de l'acide phénylacétique, caractérisé en ce que l'on cultive un micro-organisme en présence d'acide phénylacétique, on hydroxyle l'acide phénylacétique en position ortho et on ne laisse pas se poursuivre la réaction de l'acide 2-hydroxyphénylacétique.

2. Procédé suivant la revendication 1, caractérisé en ce que l'on utilise, à titre de micro-organisme, un champignon du genre Humicola ou Chaetomium.

3. Procédé suivant la revendication 1, caractérisé en ce que l'on utilise, à titre de micro-organisme, le champignon DSM 7047.

4. Champignon du genre Humicola ou Chaetomium déposé sous le numéro de dépôt DSM 7047.

5. Utilisation d'un champignon suivant la revendication 4 en vue de l'hydroxylation de composés aromatiques.
